# EUROPÄISCHE PATENTSCHRIFT

(11) **EP 0 256 302 B1**
(45) Veröffentlichungstag und Bekanntmachung des Hinweises auf die Patenterteilung: **03.02.1993**
(21) Anmeldenummer: 87110027.7
(22) Anmeldetag: 11.07.1987
(51) Int. Cl.: C07K 15/00, C12N 15/85

(54) **Verfahren zur Herstellung von humanem Antithrombin-III (ATIII) und dafür geeignete Vektoren**
Process for the production of human antithrombin III (ATIII) and vectors suitable therefor
Procédé de production d'antithrombine III (ATIII) humaine et les vecteurs appropriés

(30) Priorität: 19.07.1986 DE 3624453
(43) Veröffentlichungstag der Anmeldung: 24.02.1988
(73) Patentinhaber: BEHRINGWERKE Aktiengesellschaft, 35001 Marburg (DE)
(72) Erfinder: Zettmeissl, Gerd, Dr., D-3551 Lahntal (DE); Bröker, Michael, Dr., D-3550 Marburg (DE); Ragg, Hermann, Dr., D-6233 Kelkheim (Taunus) (DE)

(56) Entgegenhaltungen:
- EP-A- 0 090 505
- EP-A- 0 224 811
- WO-A-83/03259
- GB-A- 2 064 545
- NATURE, Band 294, 19. November 1981, Seiten 228-232, Macmillan Journals Ltd; F. LEE et al.: "Glucocorticoids regulate expression of dihydrofolate reductase cDNA in mouse mammary tumour virus chimaeric plasmids"
- MOLECULAR AND CELLULAR BIOLOGY, Band 5, Nr. 7, Juli 1985, Seiten 1750-1759, American Society for Microbiology; R.J. KAUFMAN et al.: "Coamplification and coexpression of human tissue-type plasminogen activator and murine dihydrofolate reductase sequences in Chinese hamster ovary cells"
- THE JOURNAL OF BIOLOGICAL CHEMISTRY, Band 259, Nr. 24, 25. Dezember 1984, Seiten 15386-15392, The American Society of Biological Chemists, Inc., US; E.V. PROCHOWNIK et al.: "In vivo transcription of a human antithrombin III "Minigene""
- BIOTECHNOLOGY, Band 5, Nr. 7, Juli 1987, Seiten 720-725; C. ZETTLMEISSL et al.: "Expression of biologically active human antithrombin III in Chinese hamster ovary cells"

## Beschreibung

Aus der europäischen Patentanmeldung mit der Veröffentlichungs-Nr. 0 090 505 ist die Herstellung und Charakterisierung der für ATIII kodierenden cDNA bekannt sowie die Herstellung eines humanen ATIII in E. coli. In dieser Anmeldung finden sich auch Angaben, wie man ATIII in Säugerzellkulturen herstellen kann. Als geeigneter Vektor wird ein pBR322-Derivat angeführt, das einen Marker zur Selektion in E. coli sowie einen E. coli-Replikationsursprung enthält. Der Vektor soll weiterhin den SV40-Replikationsursprung (abgeleitet von einem 342bp PvuII-HindIII-Fragment) enthalten.

Demgegenüber betrifft die vorliegende Erfindung ein besonders vorteilhaftes Verfahren, bei dem als Wirtszelle eine Säugerzelle dient, die nicht oder nicht in ausreichendem Maß Dihydrofolatreduktase produzieren kann, und wobei diese Wirtszelle mit zwei Expressionsvektoren kotransfiziert wird, von denen der eine das ATIII-Gen trägt und der andere die Produktion der Dihydrofolatreduktase bewirkt.

Die Erfindung betrifft somit ein Verfahren zur Herstellung von ATIII in Säugerzellkultur mit Hilfe eines Expressionsvektors, der die ATIII-cDNA enthält, das dadurch gekennzeichnet ist, daß als Säugerzelle eine dhfr⁻-Zelle dient, die außer dem genannten Vektor mit einem weiteren Vektor kotransfiziert wird, der ein DHFR-Gen trägt welches durch einen schwachen promoter kontrolliert wird. Weitere Aspekte der Erfindung und ihre bevorzugten Ausgestaltungen werden im folgenden näher erläutert bzw. in den Patentansprüchen definiert.

Das erfindungsgemäße Kotransfektionssystem benutzt also zwei Expressionsvektoren, wobei der erste ein beliebiger Vektor, beispielsweise ein pBR322-Derivat ist, bei dem das ATIII-Gen in an sich bekannter Weise unter der Kontrolle eines in eukaryotischen Zellen wirksamen Promotors steht, während beim zweiten Vektor, der für die Dihydrofolatreduktase kodiert und damit auch eine Möglichkeit zur Genamplifikation erlaubt, die Expression des DHFR-Gens von einer DNA-Sequenz der pBR322-ori-Region kontrolliert wird.

Die Figuren zeigen beispielhaft besonders bevorzugte Ausgestaltungen der Erfindung:
In Figur 1 und ihrer Fortsetzung, Figur 1a, ist die Konstruktion des Vektors pSVA STOP 1 beschrieben, der das pBR322-EcoRI-PvuII-Fragment, das HindIII-PvuII-Fragment mit dem Replikationsursprung und dem Promotor für die frühen und späten Transkripte sowie die Polyadenylierungsstelle aus dem BamHI-HpaI-Fragment aus dem SV40-Genom und zusätzlich den Translationsstop aus dem Plasmid pUC12 STOP enthält.
Die Figur 2 zeigt dann den Einbau der für ATIII codierenden DNA-Sequenz in den Vektor pSVA STOP 1.
Die Figur 3 beschreibt die Konstruktion der Vektoren pMTVAdhfr (der den MMTV-LTR-Promotor enthält) und pSVOAdhfr (der keinen eukaryotischen bzw. viralen Promotor aufweist).
Die Figur 4 zeigt ATIII-Expressionsraten von Zellinien, die je einen der beiden letztgenannten Vektoren enthalten und über eine Genamplifikation mit Methotrexat erhalten wurden.

Zweckmäßig wird nicht nur der erste Vektor, der das ATIII-Gen enthält, sondern auch der zweite Vektor mit dem DHFR-Gen als pBR322-Derivat ausgestaltet. Solche Vektoren haben den Vorteil, daß sie in E. coli amplifiziert und gleichzeitig als "Pendelvektoren" in eukaryotischen Zellen eingesetzt werden können. Als Selektionsmarker dient vorteilhaft das Ampicillinresistenzgen, das in dem EcoRI-PvuII-pBR322-Fragment enthalten ist. Selbstverständlich können in an sich bekannter Weise andere oder weitere Marker eingebaut werden und nicht essentielle Regionen variiert oder eliminiert werden.

Eine weitere bevorzugte Ausgestaltung der Vektoren besteht darin, daß im Anschluß an die Strukturgene die Translationsstop-Sequenz aus pUC12 STOP (M. Bröker und E. Amann, Appl. Microbiol. Biotechnol. 23 (1986) 294-296) folgt. Auf diesem DNA-Segment befinden sich Translationsstop-Codons in allen drei Leserastern, was die Expression von modifizierten Genen erlaubt, die kein eigenes Translationsstopsignal besitzen.

Säugerzellen, die nicht oder nicht in ausreichendem Maß Dihydrofolatreduktase produzieren (dhfr⁻), und geeignete Vektoren, die diesen Defekt kompensieren können, sind allgemein bekannt (Ernst-L. Winnacker, Gene und Klone, eine Einführung in die Gentechnologie, VCH Verlagsgesellschaft Weinheim, 1984, S. 267/268, 282, 289). So ist beispielsweise die Isolierung von CHO-Zellmutanten, die hinsichtlich ihrer Dihydrofolatreduktase-Aktivität defizient sind, von G. Urlaub und L.A. Chasin, Proc. Natl. Acad. Sci. USA 77 (1980), 4216-4220, beschrieben. Man erhält so Zellinien vom dhfr⁻-Typ. Analog können auch andere Säugerzellinien in reproduzierbarer Weise hergestellt werden. Die Mutanten sind in dreifacher Weise auxotroph und brauchen zum Wachstum Glycin, ein Purinnucleotid wie Hypoxanthin und Thymidin.

Vektoren, die das Dihydrofolatreduktase-Gen in tierische Zellen einbringen können, sind beispielsweise von F. Lee et al., Nature 294 (1981) 288 beschrieben. Hierbei wird von der cDNA Gebrauch gemacht, die für Maus-Dihydrofolatreduktase codiert (A.C.Y. Chang et al., Nature 275 (1978) 617-624). In analoger Weise können andere in Säugerzellen funktionelle Gene für Dihydrofolatreduktase eingesetzt werden.

Bevorzugt ist das System der CHO-dhfr⁻-Zelle und des Maus-DHFR-Gens.

Bevorzugte Expressionsvektoren für ATIII nutzen den frühen Promotor aus dem DNA-Bereich des SV40-Replikationsursprungs, den humanen Metallothionein-II-Promotor (M. Karin und R.I. Richards, Nature 299 (1982) 797-802), den Enhancer-Promotor-Bereich eines "immediate early"-Gens aus HCMV (human cytomegalovirus, M. Boshart et al., Cell 41 (1985) 521-530), oder den Drosophila heat shock protein 70 (hsp 70)-Promotor (Holmgren et al., Cell 18 (1979) 1359-1370). Die Expression der Dihydrofolatreduktase kann unter der Kontrolle eines in der Säugerzellinie schwach wirksamen Promotors wie des MMTV-LTR (mouse mammary tumor virus-long terminal repeat, F. Lee et al., a.a.O.) oder - entsprechend einer besonderen Ausgestaltung der Erfindung - auch ohne eukaryotischen Promotor erfolgen. Im letzteren Fall ist dem DHFR-Gen unmittelbar eine DNA-Sequenz aus pBR322 vorgeschaltet, die offenbar in Säugerzellen als schwacher Promotor erkannt wird (K.-D. Langner et al., Proc. Natl. Acad. Sci. USA 83 (1986) 1598-1602).

Geeignete Expressionssysteme sind auch in den US-Patentschriften 4 399 216 und 4 576 821 sowie in den europäischen Patentanmeldungen mit den Veröffentlichungs-Nummern 0 100 521 und 0 117 058 bis 0 117 060 beschrieben.

Eine weitere Ausgestaltung der Erfindung besteht darin, daß Vektoren eingesetzt werden, die keine Introns bzw. "splice sites" enthalten. Es war überraschend, daß solche Vektoren so gut wirksam sind, da in der EP-A2 090 505 RNA-"splice sites" für Vektoren zur Expression der ATIII in Zellkulturen gefordert werden.

Es ist bekannt, daß die Dihydrofolatreduktase durch Methotrexat inhibiert wird. Zellen, die in Medien ohne Glycin, Hypoxanthin und Thymidin wachsen, werden deshalb durch Methotrexat in ihrem Wachstum gehemmt oder abgetötet. Durch Variation der Methotrexat-Konzentration im Medium und der Zelldichte können Zellen, die in Gegenwart von Methotrexat wachsen, selektioniert werden. Diese Zellen sind aufgrund einer Amplifikation der DHFR-Gene und einer damit verbundenen erhöhten Produktion des Enzyms DHFR resistent gegenüber der gewählten Methotrexat-Konzentration. Überlebende Zellen können erneut erhöhten Methotrexat-Konzentrationen ausgesetzt werden, was dann zu Zellinien mit einer noch größeren Zahl von DHFR-Genen führt.

Die Genamplifikation kann nach zwei verschiedenen Techniken durchgeführt werden:
1. Die Zellen werden durch einfache Passagen in steigende Methotrexat-Konzentrationen amplifiziert. Hierbei entsteht eine genetisch heterologe Zellpopulation, in der Zellen mit unterschiedlichem Amplifikationsgrad vorliegen.
2. Bei jeder Methotrexat-Konzentrationsstufe werden genetisch einheitliche Zellklone isoliert und analysiert, wobei nur die am besten exprimierenden Zellinien einer Stufe in die nächsthöhere übernommen werden.

Es hat sich gezeigt, daß bei erfindungsgemäß kotransfizierten Zellinien die Genamplifikation mit Methotrexat nicht nur zu einer Vermehrung der DHFR-Gene, sondern auch zu einer Amplifikation des ATIII-Gens führt.

Zur Vermehrung animaler Zellen in Kultur benötigt man im allgemeinen die Anwesenheit von Serum im Wachstumsmedium. Es wurde nun gefunden, daß man ATIII produzierende Zellinien absatzweise in serumhaltigem und serumfreiem Medium halten kann, wobei über mehrere Passagen kein Absinken der ATIII-Expressionsraten gefunden wurde. Diese Ausgestaltung des erfindungsgemäßen Verfahrens ist nicht nur deshalb vorteilhaft, weil teures Serum eingespart werden kann, sondern sie erleichtert auch die Abtrennung des ATIII erheblich, wenn die letzte Produktionsstufe serumfrei erfolgt.

Das erfindungsgemäß erhaltene rekombinante ATIII zeigt volle biologische Aktivität. Es wird - genauso wie aus Humanplasma isoliertes Naturprodukt - durch Heparin stimuliert. Es eignet sich deshalb wie das natürliche Produkt zur Herstellung von Arzneimitteln.

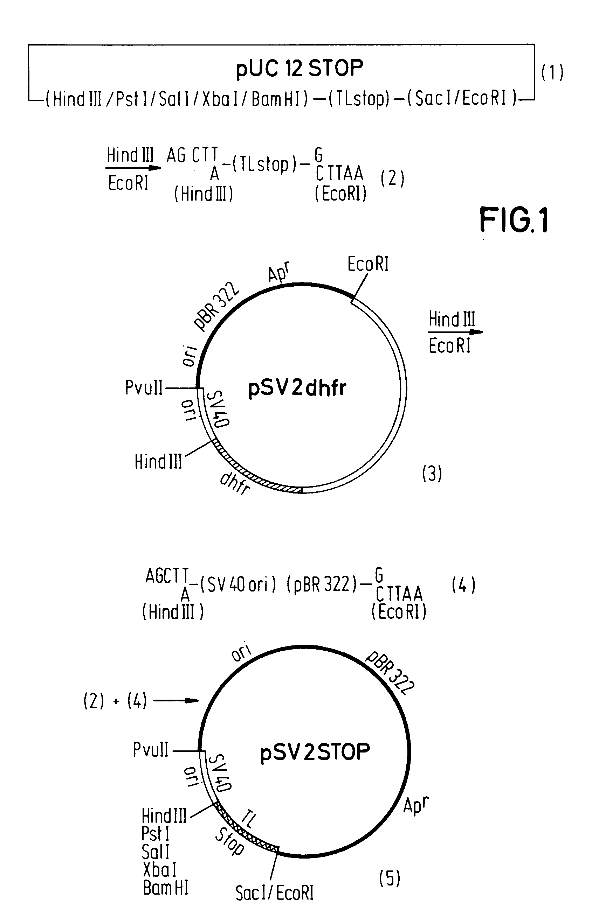

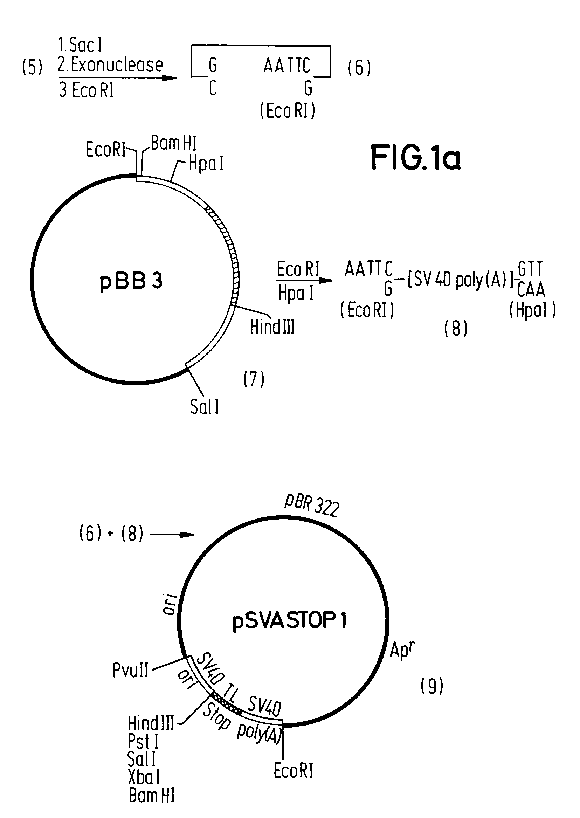

In den folgenden Beispielen wird die Erfindung näher erläutert. Die Vektorkonstruktionen werden zusätzlich durch die Figuren 1 bis 3 veranschaulicht, wobei die Zeichnungen im allgemeinen nicht maßstabsgerecht sind. Insbesondere kleine Fragmente und Polylinker Sequenzen sind in der Regel "gedehnt" dargestellt.

### Beispiel 1

### a) Konstruktion eines Expressionsvektors für animale Zellen

Das Plasmid pSV2dhfr (Fig. 1) (Lee et al., a.a.O.) wurde mit HindIII und EcoRI geschnitten und das 2,65 kb Vektorfragment, das den SV40-"early"-Promotor trägt, wurde isoliert. In den derart vorbehandelten Vektor wurde ein 67 bp HindIII-EcoRI-Fragment aus pUC12 STOP (Bröker und Amann, a.a.O.) ligiert, was zu dem Plasmid pSV2 STOP führt. Auf dem 67 bp-Fragment aus pUC12 STOP befinden sich Translationsstopcodons in allen drei Leserastern. pSV2 STOP wurde mit SacI linearisiert und der entstehende 3'-Überhang mit Hilfe der 3'→5'-Exonukleaseaktivität der DNA-Polymerase I entfernt. Anschließend wurde mit EcoRI verdaut. Nach Ligierung mit einem 133 bp großen EcoRI-HpaI-Fragment aus pBB3 (Fig. 1a) (B. Bourachot et al., EMBO J. l (1982) 895-900), das das SV40-Polyadenylierungssignal für frühe Transkripte trägt, konnte der Expressionsvektor pSVA STOP1 erhalten werden.

Die Polyadenylierungsstelle kann auch aus dem Vektor pIG6 (Bourachot et al., a.a.O.) isoliert werden. Genauso kann man aus dem SV40-Genom das 133bp BamHI-HpaI-Fragment isolieren, die BamHI-Schnittstelle auffüllen und einen EcoRI-Linker ansetzen.

pSVA STOP1 trägt somit zwischen dem SV40-"early"-Promotor und dem SV40-Polyadenylierungssignal für frühe Transkripte einen Klonierungspolylinker mit drei singulären Restriktionsstellen (HindIII-SalI-XbaI) und eine Sequenz mit Translationsstops in allen drei Leserastern.

### b) Konstruktion eines AT III-Expressionsvektors

Die ATIII-cDNA ist aus der EP-A2 0 090 505 bekannt und kann wie dort beschrieben hergestellt oder nach üblichen Verfahren synthetisiert werden. In der deutschen Patentanmeldung P 36 18 638.4 ist das Plasmid pßAT6 (Figur 1) vorgeschlagen, das die ATIII-cDNA in der SmaI-Schnittstelle des Plasmid pUC13 enthält.

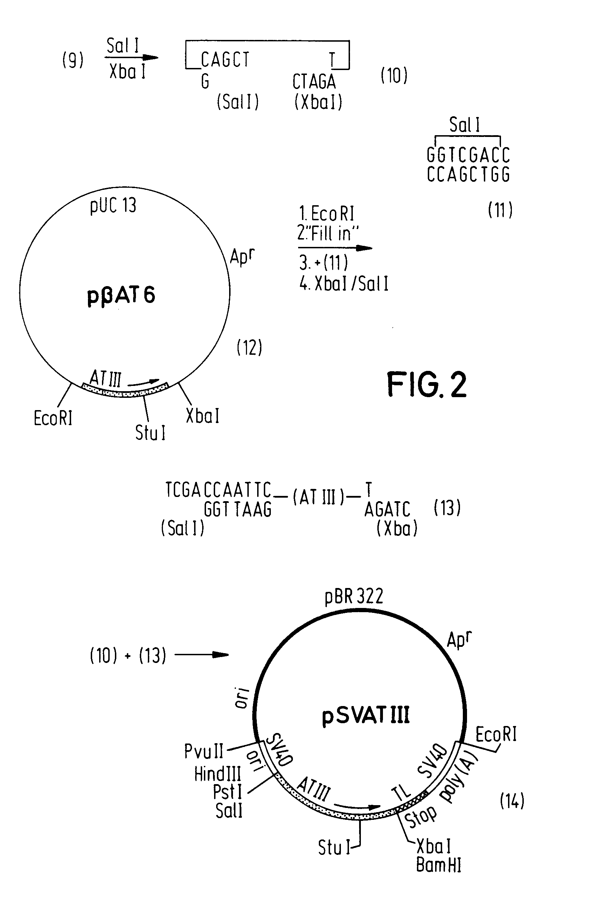

Das Subklonierungsplasmid für ATIII-cDNA, pßAT6 (Fig. 2), wurde durch Schneiden an der singulären EcoRI-Stelle linearisiert. Der entstehende 5'-Überhang wurde durch Auffüllen des Gegenstrangs mit DNA-Polymerase I (Klenow-Fragment) stumpfendig gemacht und anschließend mit dem SalI-Linker
5'd(pGGTCGACC) 3'
ligiert. Durch darauffolgende XbaI-Verdauung konnte ein etwa 1400 bp großes DNA-Fragment isoliert werden, das für das vollständige Prae-ATIII kodiert. pSVATIII wurde durch Ligieren dieses Fragments in den mit SalI und XbaI geschnittenen Expressionsvektor pSVASTOP1 erhalten. Die ATIII-Transkriptionseinheit auf pSVATIII besitzt keine m-RNA-"splice sites".

### c) Konstruktion von DHFR-Expressionsvektoren zur Kontransfektion

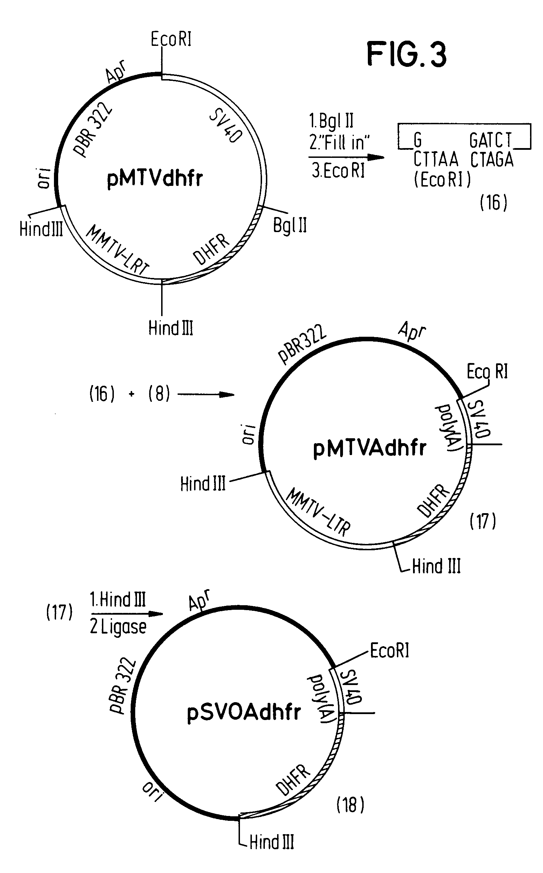

Ausgangspunkt für die zur Kotransfektion verwendeten DHFR-Vektoren war das Plasmid pMTVdhfr (Lee et al., a.a.O.). pMTVdhfr (Fig. 3) wurde mit BglII geschnitten und die überstehenden 5'-Enden der DNA mit Hilfe der DNA-Polymerase I (Klenow-Fragment) aufgefüllt. Nach Verdauung mit EcoRI wurde ein 4,47 kb großes Fragment isoliert und mit einem 133 bp EcoRI-HpaI-Fragment aus pBB3 (Bourachot et al., a.a.O.) ligiert. Das neue Plasmid pMTVAdhfr trägt die Maus-DHFR-cDNA, flankiert vom MMTV-LTR und der SV40-Polyadenylierungsstelle für frühe Transkripte.

pSVOAdhfr wurde aus pMTVAdhfr durch Deletion eines 1450 bp großen HindIII-Fragmentes erhalten, das den MMTV-LTR trägt.

Weder pMTVAdhfr noch pSVOAdhfr besitzen m-RNA-"splice sites".

### Beispiel 2

Die Plasmide pSVATIII und pMTVAdhfr bzw. pSVOAdhfr wurden mit Hilfe der Calciumphosphatpräzipitationsmethode (Graham und von der Eb, Virology 52 (1973) 456-467) in CHO-dhfr⁻-Zellen kotransfiziert. 20 µg des Plasmids pSVATIII wurden hierzu mit 5 µg der DHFR-Expressionsplasmide pMTVAdhfr oder pSVOAdhfr gemischt und kopräzipitiert. Das Kopräzipitat der Plasmidmischung wurde, wie oben beschrieben (0,5 x 10⁶ Zellen in 25 cm² Kulturflasche), in CHO-dhfr -Zellen transfiziert. Nach 3 Tagen wurden die Zellen trypsiniert, in mehrere 60 mm Petrischalen übertragen und mit Selektionsmedium (ohne Glycin, Hypoxanthin und Thymidin) versetzt. Unter diesen Bedingungen überleben nur die Zellen, die stabil mit dem DHFR-Gen transfiziert worden sind.

Nach 1-3 Wochen werden Kolonien, bestehend aus transfizierten Zellen, in den Petrischalen sichtbar. Folgende Transfektionsraten konnten hierbei erzielt werden:

| | |
|---|---|
| pMTVAdhfr | 5 x 10⁻⁶ |
| pSVOAdhfr | 1 x 10⁻⁵ |

Einzelne Kolonien wurden isoliert und in Medium ohne Glycin, Hypoxanthin und Thymidin vermehrt.

Kulturüberstände dieser neuen Zellinien (CHO SVAT III) wurden mittels eines spezifischen ELISAs zum Nachweis von menschlichem ATIII auf rekombinantes ATIII getestet. Hierbei wurde gefunden, daß jeweils 20 % der getesteten CHO-dhfr⁺-Zellinien nachweisbar menschliches ATIII ins Medium sezernieren. Um die Expressionsrate der ATIII produzierenden Linien quantitativ zu bestimmen, wurde folgende Standardtestprozedur durchgeführt: 0,5 x 10⁶ Zellen wurden in 5 ml Medium in 25 cm² Kulturflaschen ausplattiert. Nach 24 h wurde das Medium gewechselt (5 ml). Weitere 24 h später wurde das Medium für den ELISA gesammelt und die Zellen trypsiniert und gezählt. Die angegebenen Werte stellen Mittelwerte über mindestens drei Passagen dar, wobei die Expressionsraten innerhalb einer Schwankung von ±25 % konstant bleiben. Bei allen im folgenden angegebenen Expressionsraten (µg/10⁶ Zellen/24 h) lag die Zellzahl/25 cm² Flasche am Ende des Tests bei 1±0,25 x 10⁶ Zellen. Die folgende Übersicht gibt die Expressionsraten der in beschriebener Art und Weise getesteten Basisklone an:

| | |
|---|---|
| CHO SVAT III-MTVAdhfr | Klon 1: 0,14 µg/10⁶ Zellen/24 h |
| | Klon 2: 0,11 µg/10⁶ Zellen/24 h |
| CHO SVAT III-SVOAdhfr | Klon 1: 0,08 µg/10⁶ Zellen/24 h |
| | Klon 2: 0,14 µg/10⁶ Zellen/24 h |

### Beispiel 3

### Amplifikation der integrierten ATIII-Sequenzen

### a) Amplifikation ohne Isolierung von Einzelklonen

CHO SVAT III-MTVAdhfr (Klon 1 und 2) und CHO SVAT-III-SVOAdhfr (Klon 1 und 2) wurden sukzessive in steigende Methotrexat-(Mtx)-Konzentration umgesetzt. Nach der oben beschriebenen Standardmethode wurden folgende Expressionsraten (µg/10⁶ Zellen/24 h) bestimmt:

| Mtx | CHO SVAT III-pMTVAdhfr | | CHO SVAT III-SVOAdhfr | |
|---|---|---|---|---|
| | Klon 1 | Klon 2 | Klon 1 | Klon 2 |
| 0 | 0,14 | 0,11 | 0,08 | 0,14 |
| 0,05 | 0,34 | 0,54 | - | - |
| 0,15 | - | 0,60 | 0,64 | 2,7 |
| 0,3 | 0,55 | 1,15 | - | 3,3 |
| 0,6 | 1,18 | 2,00 | 0,86 | - |
| 1 | - | - | 1,5 | - |
| 5 µM | - | 4,00 | - | 8,5 |

### b) Amplifikation unter Isolierung von Einzelklonen

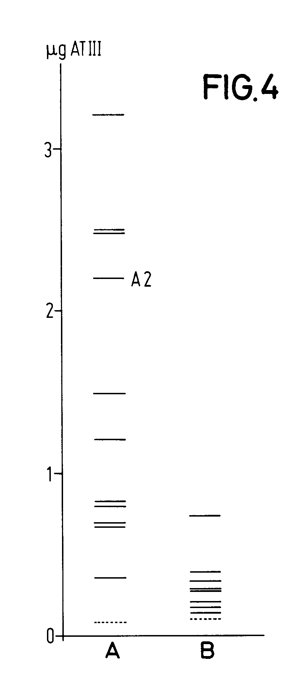

Als Mtx-Konzentrationsstufen wurden 0,1 µM und 1 µM eingesetzt. Ausgehend von CHO SVAT III-MTVAdhfr (Klon 2) wurden 8 ("B" in Fig. 4) und ausgehend von CHO SVAT III-SVOAdhfr (Klon 1) 11 ("A" in Fig. 4) gegenüber 0,1 µM Mtx resistente Klone isoliert und bezüglich ihrer Expressionsraten charakterisiert (Fig. 4); die Ordinate zeigt die Ausbeute an ATIII in µg/10⁶ Zellen/24 h. Alle diese Klone produzieren eine größere Menge ATIII als der entsprechende Ausgangsklon (s.o.). Die Expressionsraten der bei 0,1 µM Mtx wachsenden Klone liegen ausgehend von CHO SVAT III-MTVAdhfr (Klon 2) zwischen 0,15 und 0,8 µg/10⁶ Zellen/24 h (entsprechend einer Erhöhung um den Faktor 1,4 - 7) und ausgehend von CHO SVAT III-SVOAdhfr (Klon 1) zwischen 0,35 und 3,2 µg/10⁶ Zellen/24 h (entsprechend einer Erhöhung um den Faktor 4 - 40). In "Southern blots" (Southern, J.Mol.Biol. 98 (1975) 503) konnte gezeigt werden, daß in allen analysierten bei 0,1 µM Mtx wachsenden Klonen ein spezfisches 1400 bp großes BamHI-HindIII-Fragment aus der transfizierten ATIII-cDNA amplifiziert wird.

Mit einer der am besten produzierenden bei 0,1 µM Mtx resistenten Zellinien (CHO SV AT III-SVOAdhfr, Klon 1 A2; 2,2 µg/10⁶ Zellen/24 h; in Fig. 4 als "A2" eingezeichnet) wurde eine weitere Amplifikationsrunde bei 1 µM Mtx durchgeführt. Hierbei konnte bei wiederum 9 getesteten Klonen eine Zellinie (CHO SVAT III-SVOAdhfr Klon 1A27) isoliert werden, die ca. 10µg/10⁶ Zellen/24 h produziert.

### Beispiel 4

### Synthese von ATIII in serumfreien Medium

Der CHO SVAT III-SVOAdhfr Klon 1 A27 (1 µM Mtx) wurde in 175 cm² Kulturflaschen in HamF12 Selektionsmedium (ohne Glycin, Thymidin und Hypoxanthin) in Anwesenheit von 10 % (v/v) foetalem Kälberserum zu etwa 80 % Konfluenz angezüchtet, die Zellen wurden anschließend mit serumfreiem Iskoves-Medium gewaschen und dann für 48 h in diesem Medium zur Produktion von ATIII gehalten. Nach diesem Zeitraum lag ATIII in einer Konzentration von 5 - 6 µg/ml im Medium vor. Die Zellen wurden daraufhin für 24 - 48 h in serumhaltigem Medium gehalten, bis eine weitere 48 h dauernde Produktionsrunde in serumfreiem Medium begann. Der beschriebene Produktionszyklus konnte mehrmals ohne Absinken der ATIII-Expressionsraten wiederholt werden.

### Beispiel 5

### Reinigung und Charakterisierung von ATIII aus animalen Zellen

ATIII wurde mittels Affinitätschromatographie an Heparin-SEPHAROSE^{(R)} (Miller-Anderssen et al., Thromb. Res. 5 (1974) 439 - 452) sowohl aus serumhaltigen als auch aus serumfreien Medien angereichert. Das angereicherte Material zeigte im Immundiffusionstest nach Ouchterlony (Prog. Allergy 5, (1958) l) immunologische Identität mit aus Humanserum gewonnenem ATIII. Auch im "Western blot" mit ATIII-spezifischen Antikörpern lag Identität zwischen dem aus Humanplasma und dem aus CHO-Zellen gewonnenen rekombinanten ATIII vor.

Das von CHO-Zellen sezernierte AT III besaß sowohl Heparinkofaktor (Hensen und Loeliger, Thromb. Diath. haemah. 9 (1963) Suppl. 1, 18 - 29) als auch Progressivinhibitor-Aktivität (Schrader et al., Ärztl. Lab. 29 (1983) 35-39) in vollem Ausmaß.

## Patentansprüche (Patentansprüche für folgende(n) Vertragsstaat(en): BE, CH, DE, FR, GB, IT LI, LU, NL, SE)

1. Verfahren zur Herstellung von menschlichem Antithrombin III (ATIII) in Säugerzellkultur mit Hilfe eines Expressionsvektors, der die ATIII-cDNA enthält, dadurch gekennzeichnet, daß als Säugerzelle eine dhfr⁻-Zelle dient, die mit einem weiteren Vektor kontransfiziert wird, der ein DHFR-Gen trägt, welches durch einen schwachen Promotor kontrolliert wird.

2. Verfahren nach Anspruch 1, dadurch gekennzeichnet, daß dem DHFR-Gen kein eukaryotischer oder viraler Promotor vorgeschaltet ist.

3. Verfahren nach Anspruch 1 oder 2, dadurch gekennzeichnet daß das DHFR-Gen stromabwärts an die pBR322-ori-Region anschließt.

4. Verfahren nach Anspruch 1, 2 oder 3, dadurch gekennzeichnet, daß das DHFR-Gen stromabwärts an die ori-Region eines pBR322-Segments anschließt, das einen Selektionsmarker enthält.

5. Verfahren nach Anspruch 4, dadurch gekennzeichnet, daß der Selektionsmarker das Ampicillinresistenzgen ist.

6. Verfahren nach Anspruch 4 oder 5, dadurch gekennzeichnet, daß als pBR322-Segment das EcoRI-PvuII-Fragment eingesetzt wird.

7. Verfahren nach einem oder mehreren der vorhergehenden Ansprüche, dadurch gekennzeichnet, daß das ATIII-Gen unter der Kontrolle eines viralen Promotors steht.

8. Verfahren nach einem oder mehreren der vorhergehenden Ansprüche, dadurch gekennzeichnet, daß die Vektoren keine "splice site" enthalten.

9. Verfahren nach einem oder mehreren der vorhergehenden Ansprüche, dadurch gekennzeichnet, daß das DHFR-Gen zusammen mit dem ATIII-Gen mit Methotrexat amplifiziert wird.

10. Verfahren nach einem oder mehreren der vorhergehenden Ansprüche, dadurch gekennzeichnet, daß die ATIII-Produktion in serumfreiem Medium erfolgt.

11. Expressionsvektor mit pBR322-Sequenzen und der ATIII-cDNA, gekennzeichnet durch eine SV40-Promotorsequenz, eine SV40-Polyadenylierungsstelle und die Abwesenheit von "splice sites".

12. Vektor nach Anspruch 11, dadurch gekennzeichnet, daß die Polyadenylierungsstelle im wesentlich das 133bp BamHI-HpaI-Fragment ist.

13. Vektor nach Anspruch 11 oder 12, gekennzeichnet durch die Translationsstopsequenz aus pUC12 STOP.

14. Plasmide pSV2 STOP (Figur 1) und pSVA STOP1 (Figur 1a).

15. Plasmid pSVATIII (Figur 2).

16. Vektor mit dem Maus-DHFR-Gen, dadurch gekennzeichnet, daß das DHFR-Gen durch einen schwachen Promotor kontrolliert wird und der Vektor keine "splice site" enthält.

17. Vektor nach Anspruch 16, gekennzeichnet durch eine SV40-Polyadenylierungsstelle.

18. Plasmide pMTVAdhfr und pSVOAdhfr (Figur 3).

19. Säugerzelle, kotransfiziert einerseits mit einem Vektor nach Anspruch 11 bis 15 und andererseits mit einem Vektor nach Anspruch 16 bis 18.

20. Zelle nach Anspruch 20, selektioniert mit Hilfe von Methotrexat.

## Patentansprüche (Patentansprüche für folgende(n) Vertragsstaat(en): AT, ES, GR)

1. Verfahren zur Herstellung von menschlichem Antithrombin III (ATIII) in Säugerzellkultur mit Hilfe eines Expressionsvektors, der die ATIII-cDNA enthält, dadurch gekennzeichnet, daß als Säugerzelle eine dhfr⁻-Zelle dient, die mit einem weiteren Vektor kotransfiziert wird, der ein DHFR-Gen trägt welches durch einen schwachen promoter kontrolliert wird.

2. Verfahren nach Anspruch 1, dadurch gekennzeichnet, daß dem DHFR-Gen kein eukaryotischer oder viraler Promotor vorgeschaltet ist.

3. Verfahren nach Anspruch 1 oder 2, dadurch gekennzeichnet, daß das DHFR-Gen stromabwärts an die pBR-322-ori-Region anschließt.

4. Verfahren nach Anspruch 1, 2 oder 3, dadurch gekennzeichnet, daß das DHFR-Gen stromabwärts an die ori-Region eines pBR322-Segments anschließt, das einen Selektionsmarker enthält.

5. Verfahren nach Anspruch 4, dadurch gekennzeichnet, daß der Selektionsmarker das Ampicillinresistenzgen ist.

6. Verfahren nach Anspruch 4 oder 5, dadurch gekennzeichnet, daß als pBR322-Segment das EcoRI-PvuII-Fragment eingesetzt wird.

7. Verfahren nach einem oder mehreren der vorhergehenden Ansprüche, dadurch gekennzeichnet, daß das ATIII-Gen unter der Kontrolle eines viralen Promotors steht.

8. Verfahren nach einem oder mehreren der vorhergehenden Ansprüche, dadurch gekennzeichnet, daß die Vektoren keine "splice site" enthalten.

9. Verfahren nach einem oder mehreren der vorhergehenden Ansprüche, dadurch gekennzeichnet, daß das DHFR-Gen zusammen mit dem ATIII-Gen mit Methotrexat amplifiziert wird.

10. Verfahren nach einem oder mehreren der vorhergehenden Ansprüche, dadurch gekennzeichnet, daß die ATIII-Produktion in serumfreiem Medium erfolgt.

11. Verfahren nach Anspruch 1, dadurch gekennzeichnet, daß ein Expressionsvektor mit pBr322-Sequenzen und der ATIII-cDNA, einer SV40-Promotorsequenz, eine SV40-Polyadenylierungsstelle und ohne "splice sites" eingesetzt wird.

12. Verfahren nach Anspruch 11, dadurch gekennzeichnet, da die Polyadenylierungsstelle im wesentlichen das 113 bp BamHI-HpaI-Fragment ist.

13. Verfahren nach Anspruch 11 oder 12, dadurch gekennzeichnet, daß der Expressionsvektor die Translationsstopsequenz aus pUC12 STOP enthält.

14. Verfahren nach Anspruch 1, dadurch gekennzeichnet, daß die Plasmide pSV2 STOP (Figur 1) oder pSVA, STOP 1 (Fig. 1a) eingesetzt werden.

15. Verfahren nach Anspruch 1, dadurch gekennzeichnet, daß das Plasmid pSV ATIII (Figur 2) eingesetzt wird.

16. Verfahren nach Anspruch 1, dadurch gekennzeichnet, daß die Plasmide pMTVAdhfr oder pSVOAdhfr (Figur 3) eingesetzt werden.

17. Verfahren nach Anspruch 1, dadurch gekennzeichnet, daß Säugerzellen eingesetzt werden, die mit einem Vektor der Ansprüche 11-15 einerseits und einem Vektor der Ansprüche 2, 3, 4, 16 andererseits Kotransfiziert sind.

18. Verfahren nach Anspruch 17, dadurch gekennzeichnet, daß mit Hilfe von Methotrexat selektioniert wurde.

## Claims (Claims for the following Contracting State(s): BE, CH, DE, FR, GB, IT, LI, LU, NL, SE)

1. A process for the preparation of human antithrombin III (ATIII) in mammalian cell culture using an expression vector which contains the ATIII cDNA, which comprises use as the mammalian cell of a dhfr⁻ cell which is cotransfected with another vector which carries a DHFR gene which is controlled by a weak promoter.

2. The process as claimed in claim 1, wherein no eukaryotic or viral promoter is located upstream of the DHFR gene.

3. The process as claimed in claim 1 or 2, wherein the DHFR gene is connected downstream to the pBR322 ori region.

4. The process as claimed in claim 1, 2 or 3, wherein the DHFR gene is connected downstream to the ori region of a pBR322 segment which contains a selection marker.

5. The process as claimed in claim 4, wherein the selection marker is the ampicillin-resistance gene.

6. The process as claimed in claim 4 or 5, wherein the EcoRI-PvuII fragment is used as the pBR322 segment.

7. The process as claimed in one or more of the preceding claims, wherein the ATIII gene is under the control of a viral promoter.

8. The process as claimed in one or more of the preceding claims, wherein the vectors contain no splice site.

9. The process as claimed in one or more of the preceding claims, wherein the DHFR gene is amplified together with the ATIII gene using methotrexate.

10. The process as claimed in one or more of the preceding claims, wherein the ATIII production is carried out in serum-free medium.

11. An expression vector with pBR322 sequences and the ATIII cDNA, which contains a SV40 promoter sequence and a SV40 polyadenylation site and no splice sites.

12. A vector as claimed in claim 11, wherein the polyadenylation site is essentially the 133 bp BamHI-HpaI fragment.

13. A vector as claimed in claim 11 or 12, which contains the translation stop sequence from pUC12 STOP.

14. Plasmids pSV2 STOP (Figure 1) and pSVA STOP1 (Figure 1a).

15. Plasmid pSVATIII (Figure 2).

16. A vector with the mouse DHFR gene, in which the DHFR gene is controlled by a weak promoter, and the vector contains no splice site.

17. A vector as claimed in claim 16, which has a SV40 polyadenylation site.

18. Plasmids pMTVAdhfr and pSVOAdhfr (Figure 3).

19. A mammalian cell cotransfected with a vector as claimed in claims 11 to 15, on the one hand, and with a vector as claimed in claims 16 to 18, on the other hand.

20. A cell as claimed in claim 19, which has been selected using methotrexate.

## Claims (Claims for the following Contracting State(s): AT, ES, GR)

1. A process for the preparation of human antithrombin III (ATIII) in mammalian cell culture using an expression vector which contains the ATIII cDNA, which comprises use as the mammalian cell of a dhfr⁻ cell which is cotransfected with another vector which carries a DHFR gene which is controlled by a weak promoter.

2. The process as claimed in claim 1, wherein no eukaryotic or viral promoter is located upstream of the DHFR gene.

3. The process as claimed in claim 1 or 2, wherein the DHFR gene is connected downstream to the pBR322 ori region.

4. The process as claimed in claim 1, 2 or 3, wherein the DHFR gene is connected downstream to the ori region of a pBR322 segment which contains a selection marker.

5. The process as claimed in claim 4, wherein the selection marker is the ampicillin-resistance gene.

6. The process as claimed in claim 4 or 5, wherein the EcoRI-PvuII fragment is used as the pBR322 segment.

7. The process as claimed in one or more of the preceding claims, wherein the ATIII gene is under the control of a viral promoter.

8. The process as claimed in one or more of the preceding claims, wherein the vectors contain no splice site.

9. The process as claimed in one or more of the preceding claims, wherein the DHFR gene is amplified together with the ATIII gene using methotrexate.

10. The process as claimed in one or more of the preceding claims, wherein the ATIII production is carried out in a serum-free medium.

11. The process as claimed in claim 1, which comprises employing an expression vector with pBR322 sequences and the ATIII cDNA, with a SV40 promoter sequence, a SV40 polyadenylation site and no splice sites.

12. The process as claimed in claim 11, wherein the poly-adenylation site is essentially the 133 bp BamHI-HpaI fragment.

13. The process as claimed in claim 11 or 12, wherein the expression vector contains the translation stop sequence from pUC12 STOP.

14. The process as claimed in claim 1, which comprises employing one of the plasmids pSV2 STOP (Figure 1) or pSVA STOP1 (Figure 1a).

15. The process as claimed in claim 1, which comprises employing the plasmid pSVATIII (Figure 2).

16. The process as claimed in claim 1, which comprises employing one of the plasmids pMTVAdhfr or pSVOAdhfr (Figure 3).

17. The process as claimed in claim 1, which comprises employing mammalian cells cotransfected with a vector of claims 11 to 15, on the one hand, and with a vector of claims 2, 3, 4 and 16, on the other hand.

18. The process as claimed in claim 17, wherein selection has been carried out using methotrexate.

## Revendications (Revendications pour l'(les) Etat(s) contractant(s) suivant(s): BE, CH, DE, FR, GB, IT, LI, LU, NL, SE)

1. Procédé pour la production d'antithrombine III (ATIII) humaine en culture de cellules mammaliennes, à l'aide d'un vecteur d'expression qui contient l'ADNc d'ATIII, caractérisé en ce que, en tant que cellule mammalienne, on utilise une cellule dhfr⁻ qui est co-transfectée avec un autre vecteur qui porte un gène DHFR qui est contrôlé par un promoteur faible.

2. Procédé selon la revendication 1, caractérisé en ce qu'aucun promoteur viral ou d'eucaryote n'est disposé avant le gène DHFR.

3. Procédé selon la revendication 1 ou 2, caractérisé en ce que le gène DHFR fait suite en aval à la région ori de pBR322.

4. Procédé selon la revendication 1, 2 ou 3, caractérisé en ce que le gène DHFR fait suite en aval à la région ori d'un segment de pBR322 qui contient un marqueur de sélection.

5. Procédé selon la revendication 4, caractérisé en ce que le marqueur de sélection est le gène de résistance à l'ampicilline.

6. Procédé selon la revendication 4 ou 5, caractérisé en ce que, comme segment de pBR322, on utilise le fragment EcoRI-PvuII.

7. Procédé selon une ou plusieurs des revendications précédentes, caractérisé en ce que le gène ATIII est sous le contrôle d'un promoteur viral.

8. Procédé selon une ou plusieurs des revendications précédentes, caractérisé en ce que les vecteurs ne contiennent pas de "site d'épissage".

9. Procédé selon une ou plusieurs des revendications précédentes, caractérisé en ce que le gène DHFR est amplifié, conjointement avec le gène ATIII, à l'aide de méthotrexate.

10. Procédé selon une ou plusieurs des revendications précédentes, caractérisé en ce que la production d'ATIII s'effectue en milieu exempt de sérum.

11. Vecteur d'expression comportant des séquences de pBR322 et l'ADNc d'ATIII, caractérisé par une séquence de promoteur de SV40, un site de polyadénylation de SV40 et l'absence de "sites d'épissage".

12. Vecteur selon la revendication 11, caractérisé en ce que le site de polyadénylation est essentiellement le fragment BamHI-HpaI de 133 pb.

13. Vecteur selon la revendication 11 ou 12, caractérisé par la séquence d'arrêt de traduction provenant de pUC12 STOP.

14. Plasmides pSV2 STOP (figure 1) et pSVA STOP1 (figure la).

15. Plasmide pSVATIII (figure 2).

16. Vecteur comportant le gène DHFR murin, caractérisé en ce que le gène DHFR est contrôlé par un promoteur faible et le vecteur ne contient pas de "site d'épissage".

17. Vecteur selon la revendication 16, caractérisé par un site de polyadénylation de SV40.

18. Plasmides pMTVAdhfr et pSVOAdhfr (figure 3).

19. Cellule mammalienne co-transfectée, d'une part, par un vecteur selon les revendications 11 à 15 et, d'autre part, par un vecteur selon les revendications 16 à 18.

20. Cellule selon la revendication 19, sélectionnée à l'aide de méthotrexate.

## Revendications (Revendications pour l'(les) Etat(s) contractant(s) suivant(s): AT, ES, GR)

1. Procédé pour la production d'antithrombine III (ATIII) humaine en culture de cellules marmaliennes, à l'aide d'un vecteur d'expression qui contient l'ADNc d'ATIII, caractérisé en ce que, en tant que cellule mammalienne, on utilise une cellule dhfr⁻ qui est co-transfectée avec un autre vecteur qui porte un gène DHFR qui est contrôlé par un promoteur faible.

2. Procédé selon la revendication 1, caractérisé en ce qu'aucun promoteur viral ou d'eucaryote n'est disposé avant le gène DHFR.

3. Procédé selon la revendication 1 ou 2, caractérisé en ce que le gène DHFR fait suite en aval à la région ori de pBR322.

4. Procédé selon la revendication 1, 2 ou 3, caractérisé en ce que le gène DHFR fait suite en aval à la région ori d'un segment de pBR322 qui contient un marqueur de sélection.

5. Procédé selon la revendication 4, caractérisé en ce que le marqueur de sélection est le gène de résistance à l'ampicilline.

6. Procédé selon la revendication 4 ou 5, caractérisé en ce que, comme segment de pBR322, on utilise le fragment EcoRI-PvuII.

7. Procédé selon une ou plusieurs des revendications précédentes, caractérisé en ce que le gène ATIII est sous le contrôle d'un promoteur viral.

8. Procédé selon une ou plusieurs des revendications précédentes, caractérisé en ce que les vecteurs ne contiennent pas de "site d'épissage".

9. Procédé selon une ou plusieurs des revendications précédentes, caractérisé en ce que le gène DHFR est amplifié, conjointement avec le gène ATIII, à l'aide de méthotrexate.

10. Procédé selon une ou plusieurs des revendications précédentes, caractérisé en ce que la production d'ATIII s'effectue en milieu exempt de sérum.

11. Procédé selon la revendication 1, caractérisé en ce que l'on utilise un vecteur d'expression comportant des séquences de pBR322 et l'ADNc d'ATIII, une séquence de promoteur de SV40, un site de polyadénylation de SV40 et dépourvu de "sites d'épissage".

12. Procédé selon la revendication 11, caractérisé en ce que le site de polyadénylation est essentiellement le fragment BamHI-HpaI de 133 pb.

13. Procédé selon la revendication 11 ou 12, caractérisé en ce que le vecteur d'expression contient la séquence d'arrêt de traduction provenant de pUC12 STOP.

14. Procédé selon la revendication 1, caractérisé en ce que l'on utilise les plasmides pSV2 STOP (figure 1) ou pSVA STOP1 (figure 1a).

15. Procédé selon la revendication 1, caractérisé en ce que l'on utilise le plasmide pSVATIII (figure 2).

16. Procédé selon la revendication 1, caractérisé en ce que l'on utilise les plasmides pMTVAdhfr ou pSVOAdhfr (figure 3).

17. Procédé selon la revendication 1, caractérisé en ce que l'on utilise des cellules mammaliennes qui sont cotransfectées, d'une part, par un vecteur des revendications 11 à 15 et, d'autre part, par un vecteur des revendications 2, 3, 4, 16.

18. Procédé selon la revendication 17, caractérisé en ce que la sélection a été effectuée à l'aide de méthotrexate.
